# EUROPEAN PATENT APPLICATION

(11) **EP 1 949 908 A1**
(43) Date of publication of application: **30.07.2008**
(21) Application number: 08102503.3
(22) Date of filing: 07.03.2002
(51) Int. Cl.: A61K 38/26, C07K 14/605, A61P 3/10

(54) **Combined use of derivatives of GLP-1 analogs and PPAR ligands**

(30) Priority: 07.03.2001 US 800541; 08.03.2001 WO PCT/DK01/00150; 21.05.2001 DK 200100812; 07.09.2001 US 949344; 07.09.2001 DK 200101315; 11.09.2001 DK 200101322; 13.09.2001 US 951300
(62) Divisional of application: 02702232.6
(71) Applicant: NOVO NORDISK A/S, 2880 Bagsvaerd (DK)
(72) Inventor: Knudsen, Liselotte Bjerre, 2500, Valby (DK); Wassermann, Karsten, 2820, Gentofte (DK); Sturis, Jeppe, 3500, Vaerloese (DK); Brand, Christian Lehn, 3450, Alleroed (DK); Godtfredsen, Carsten Foged, 2730, Herlev (DK)

(57) **Abstract**

The present invention relates to methods for treatment and/or prevention of type 1 diabetes, type 2 diabetes, dyslipidemia, impaired glucose tolerance, insulin resistance, obesity and beta-cell apoptosis. More specifically, the methods and uses of the invention pertains to administration of a stable derivative of a GLP-1 analog in combination with administration of a non-thiazolidinedione peroxisome proliferating activated receptor (PPAR) ligand.

## Description

### FIELD OF THE INVENTION

The present invention relates to methods for treatment and/or prevention of type 1 diabetes, type 2 diabetes, dyslipidemia, impaired glucose tolerance, insulin resistance, obesity and beta-cell apoptosis. More specifically, the methods and uses of the invention pertains to administration of a stable derivative of a GLP-1 analog in combination with administration of a non-thiazolidinedione peroxisome proliferating activated receptor (PPAR) ligand.

### BACKGROUND OF THE INVENTION

Diabetes is a disorder of carbohydrate metabolism characterized by hyperglycemia and glucosuria resulting from insufficient production or utilization of insulin. Diabetes severely affects the quality of life of large parts of the populations in developed countries. Insufficient production of insulin is characterised as type 1 diabetes and insufficient utilization of insulin is type 2 diabetes.

Dyslipidemia, or abnormal levels of lipoproteins in blood plasma, is a frequent occurrence among diabetics. Dyslipidemia is typically characterized by elevated plasma triglycerides, low HDL (High Density Lipoprotein) cholesterol, normal to elevated levels of LDL (Low Density Lipoprotein) cholesterol and increased levels of small dense, LDL (Low Density Lipoprotein) particles in the blood. Dyslipidemia is one of the main contributors to the increased incidence of coronary events and deaths among diabetic subjects. Epidemiological studies have confirmed this by showing a several-fold increase in coronary deaths among diabetic subjects when compared with non-diabetic subjects. Several lipoprotein abnormalities have been described among diabetic subjects.

Insulin resistance is the diminished ability of insulin to exert its biologically action across a broad range of concentrations. In insulin resistance, the body secretes abnormally high amounts of insulin to compensate for this defect and a state of impaired glucose tolerance develops. Failing to compensate for the defective insulin action, the plasma glucose concentration inevitable rises, resulting in the clinical state of diabetes.
It is being recognised that insulin resistance and relative hyperinsulinemia have a contributory role in obesity, hypertension, atherosclerosis and type 2 diabetes. The association of insulin resistance with obesity, hypertension and angina has been described as a syndrome, Syndrome X, having insulin resistance as the common pathogenic link.

Apoptosis is an active process of cellular self-destruction that is regulated by extrinsic and intrinsic signals occurring during normal development. It is well documented that apoptosis plays a key role in regulation of pancreatic endocrine beta cells. There is increasing evidence that in adult mammals the beta-cell mass is subject to dynamic changes to adapt insulin production for maintaining euglycemia in particular conditions, such as pregnancy and obesity. The control of beta cell mass depends on a subtle balance between cell proliferation, growth and programmed cell death (apoptosis). A disruption of this balance may lead to impairment of glucose homeostasis. For example, it is noteworthy that glucose intolerance develops with aging when beta cell replication rates are reduced and human autopsy studies repeatedly showed a 40-60% reduction of beta cell mass in patients with non-insulin-dependent-diabetes mellitus compared with nondiabetic subjects. It is generally agreed that insulin resistance is an invariable accompaniment of obesity but that normoglycemia is maintained by compensatory hyperinsulinemia until the beta cells become unable to meet the increased demand for insulin, at which point type 2 diabetes begins.

Attempts to treatment of the multiple abnormalities associated with diabetes have prompted for the administration of several anti-diabetic medicaments in order to address these abnormalities in the different patients. Examples of anti-diabetic medicaments are proteins such as insulin and GLP-1, and small molecules such as insulin sensitizers, insulin secretagogues and appetite regulating compounds.

Human GLP-1 is a 37 amino acid residue peptide originating from preproglucagon which is synthesized i.a. in the L-cells in the distal ileum, in the pancreas and in the brain. GLP-1 is an important gut hormone with regulatory function in glucose metabolism and gastrointestinal secretion and metabolism. Processing of preproglucagon to give GLP-1 (7-36)amide, GLP-1 (7-37) and GLP-2 occurs mainly in the L-cells. A simple system is used to describe fragments and analogues of this peptide. Thus, for example, Gly⁸-GLP-1 (7-37) designates a fragment of GLP-1 formally derived from GLP-1 by deleting the amino acid residues Nos. 1 to 6 and substituting the naturally occurring amino acid residue in position 8 (Ala) by Gly. Similarly, Lys³⁴(N^{ε}-tetradecanoyl)-GLP-1 (7-37) designates GLP-1 (7-37) wherein the ε-amino group of the Lys residue in position 34 has been tetradecanoylated. PCT publications WO 98/08871 and WO 99/43706 disclose stable derivatives of GLP-1 analogs, which have a lipophilic substituent. These stable derivatives of GLP-1 analogs have a protracted profile of action compared to the corresponding GLP-1 analogs.

β-Aryl-α-oxosubstituted alkylcarboxylic acids (PCT publication WO 99/19313) and azolidinediones (PCT publication WO 97/41097) are insulin sensitizers useful as antidiabetic agents. Examples of these compounds are e.g. (-)-2-ethoxy-3-(4-(2-phenoxazin-10-yl-ethoxy)-phenyl)-propionic acid (PCT publication WO 00/50414) and 5-[[4-[3-methyl-4-oxo-3,4-dihydro-2-quinazolinyl]methoxy]phenyl-methyl]thiazolidine-2,4-dione (PCT publication WO 97/41097). The compounds are useful for treatment and/or prophylaxis of e.g. type 2 diabetes, impaired glucose tolerance, dyslipidemia, and obesity.

PCT publication WO 00/78333 describes co-administration of GLP-1 and thiazolidinedione for treatment of NIDDM. A side effect of thiazolidinedione was stated to be reduced and a synergistic effect of combining GLP-1 with thiazolidinedione has been alleged.

Combined treatment with derivatives of GLP-1 analogs and non-thiazolidinedione PPAR ligands convey the benefits of both compounds while reducing side effects associated with each compound. Thus, there is a need for the therapeutic benefits of the individual compounds while simultaneously reducing the side effects.

### SUMMARY OF THE INVENTION

One object of the present invention is to provide methods, which can effectively be used in the treatment or prophylaxis of type 1 diabetes, type 2 diabetes or dyslipidemia. Another object of the invention is to provide methods, which can effectively be used in the treatment or prophylaxis of impaired glucose tolerance, insulin resistance, obesity or beta-cell apoptosis.
A further object of the present invention is to provide methods for increasing the number of beta-cells in a subject, increasing the size of beta-cells in a subject or stimulating beta-cell proliferation.

The invention includes a method for the treatment of type 1 diabetes, type 2 diabetes, dyslipidemia, impaired glucose tolerance, insulin resistance, obesity and beta-cell apoptosis, which method comprises administration of an effective amount of a stable derivative of a GLP-1 analog and an effective amount of a non-thiazolidinedione PPAR ligand to a patient in need thereof.

The invention further includes a method for increasing the number of beta-cells in a subject, increasing the size of beta-cells in a subject or stimulating beta-cell proliferation, which method comprises administration of an effective amount of a stable derivative of a GLP-1 analog and an effective amount of a non-thiazolidinedione PPAR ligand to a patient in need thereof.

In one embodiment of the invention, the stable derivative of a GLP-1 analog is an analog with a lipophilic substituent, preferably Arg³⁴, Lys²⁶(N^{ε}-(γ-Glu(N^{α}-hexadecanoyl)))-GLP-1(7-37).

In another embodiment of the invention the non-thiazolidinedione PPAR ligand is an β-aryl-α-oxosubstituted alkylcarboxylic acid, preferably (-)-2-ethoxy-3-(4-(2-phenoxazin-10-yl-ethoxy)-phenyl)-propionic acid or a salt thereof.

In yet another embodiment of the invention the non-thiazolidinedione PPAR ligand and the stable derivative of a GLP-1 analog are administered in suboptimal dosages.

In yet another embodiment of the invention the non-thiazolidinedione PPAR ligand and the stable derivative of a GLP-1 analog are administered in amounts and for a sufficient time to produce a synergistic effect.

### DETAILED DESCRIPTION OF THE INVENTION

A stable derivative of a GLP-1 analog is a GLP-1 analog or a derivative thereof which exhibits an in vivo plasma elimination half-life of at least 10 hours in man, as determined by the method described below. Examples of stable derivatives of GLP-1 analogs can be found in WO 98/08871 and WO 99/43706. The method for determination of plasma elimination half-life of a compound in man is : The compound is dissolved in an isotonic buffer, pH 7.4, PBS or any other suitable buffer. The dose is injected peripherally, preferably in the abdominal or upper thigh. Blood samples for determination of active compound are taken at frequent intervals, and for a sufficient duration to cover the terminal elimination part (e.g. Pre-dose, 1, 2, 3, 4, 5, 6, 7, 8, 10, 12, 24 (day 2), 36 (day 2), 48 (day 3), 60 (day 3), 72 (day 4) and 84 (day 4) hours post dose). Determination of the concentration of active compound is performed as described in Wilken et al., Diabetologia 43(51):A143, 2000. Derived pharmacokinetic parameteres are calculated from the concentration-time data for each individual subject by use of non-compartmental methods, using the commercially available software WinNonlin Version 2.1 (Pharsight, Cary, NC, USA). The terminal elimination rate constant is estimated by log-linear regression on the terminal log-linear part of the concentration-time curve, and used for calculating the elimination half-life.

Non-thiazolidinedione PPAR ligands are a class of compounds which through their binding to peroxisome proliferating activated receptors (PPARs), such as subtypes PPAR-alpha, PPAR-gamma and PPAR-delta, work as 'lipid sensors' providing the molecular link to glycaemic control and insulin sensitization in the treatment of type 2 diabetes and dyslipidemia. Normoglycaemic effect achieved upon ligand-PPAR interaction may be mediated through regulation of fatty acid homeostasis that apparently leads to enhanced insulin action with subsequent increase of glucose utilization in peripheral tissues such as muscle and fat, and suppression of hepatic gluconeogenesis, cf., The glucose fatty acid cycle (Randle PJ, Garland PB, Hales CN, Newsholme EA. The glucose-fatty-acid cycle: its role in insulin sensitivity and the metabolic disturbances of diabetes mellitus. Lancet 1963;i:785-789.

It has been discovered that in the treatment of diabetes there is a synergistic effect of stable derivatives of GLP-1 analogs and non-thiazolidinedione PPAR ligands. A synergistic effect of two compounds is in terms of statistical analysis an effect which is greater than the additive effect which results from the sum of the effects of the two individual compounds. Treatment of Zucker Diabetic Fatty (ZDF) rats with a combination of Arg³⁴, Lys²⁶(N^{ε}-(γ-Glu(N^{α}-hexadecanoyl)))-GLP-1 (7-37) and a non-thiazolidinedione PPAR ligand was compared to the corresponding treatment of ZDF rats with Arg³⁴, Lys²⁶(N^{ε}-(γ-Glu(N^{α}-hexadecanoyl)))-GLP-1 (7-37) alone, and with non-thiazolidinedione PPAR ligand alone. Statistical analysis of the experimental results showed a significant interaction which demonstrate that combined treatment with non-thiazolidinedione PPAR ligands and Arg³⁴, Lys²⁶(N^{ε}-(γ-Glu(N^{α}-hexadecanoyl)))-GLP-1 (7-37) has profound synergistic effects on HbA_{1c} and the 24-hour plasma glucose profile.

A strong synergistic effect of two compounds permits the dosages of these compounds in the combined treatment to be below the optimal dosages of the individual compounds in single-compound treatment. Thus, these suboptimal dosages of the individual compounds reduce side effects since lower dosages are needed for the same therapeutic effect in the combined treatment.

Accordingly, the present invention relates to methods for treatment of type 1 diabetes, type 2 diabetes, dyslipidemia, impaired glucose tolerance, insulin resistance, obesity and beta-cell apoptosis. The methods comprise administration of an effective amount of a stable derivative of a GLP-1 analog and administration of an effective amount of a non-thiazolidinedione PPAR ligand. The two compounds may be co-administered or they may be administered separately as two medicaments. Furthermore, the first compound may be administered in a regimen, which additionally comprises treatment with the second compound. Hence, according to the present invention the only provision is that there must be overlapping periods of treatment with the stable derivative of a GLP-1 analog and the non-thiazolidinedione PPAR ligand.

In a second aspect the present invention relates to methods for increasing the number of beta-cells in a subject, increasing the size of beta-cells in a subject or stimulating beta-cell proliferation. The methods comprise administration of an effective amount of a stable derivative of a GLP-1 analog and an effective amount of a non-thiazolidinedione PPAR ligand to a patient in need thereof. The two compounds may be co-administered or they may be administered separately as two medicaments. Furthermore, the first compound may be administered in a regimen, which additionally comprises treatment with the second compound. Hence, according to the present invention the only provision is that there must be overlapping periods of treatment with the stable derivative of a GLP-1 analog and the non-thiazolidinedione PPAR ligand.

In one embodiment the stable derivative of a GLP-1 analog is Arg³⁴, Lys²⁶(N^{ε}-(γ-Glu(N^{α}-hexadecanoyl)))-GLP-1 (7-37). Arg³⁴, Lys²⁶(N^{ε}-(γ-Glu(N^{α}-hexadecanoyl)))-GLP-1 (7-37) is disclosed in WO 98/08871.

In another embodiment the non-thiazolidinedione PPAR ligand is a β-aryl-α-oxosubstituted alkylcarboxylic acid, or a salt thereof.

In another embodiment the non-thiazolidinedione PPAR ligand is (-)-2-ethoxy-3-(4-(2-phenoxazin-10-yl-ethoxy)-phenyl)-propionic acid, or a salt thereof. (-)-2-ethoxy-3-(4-(2-phenoxazin-10-yl-ethoxy)-phenyl)-propionic acid, or a salt thereof, is disclosed in WO 00/50414. A typically salt of this compound is the arginine salt disclosed in WO 00/63189.

In another embodiment the stable derivative of a GLP-1 analog is Arg³⁴, Lys²⁶(N^{ε}-(γ-Glu(N^{α}-hexadecanoyl)))-GLP-1(7-37) and the non-thiazolidinedione PPAR ligand is (-)-2-ethoxy-3-(4-(2-phenoxazin-10-yl-ethoxy)-phenyl)-propionic acid, or a salt thereof.

In yet another embodiment the stable derivative of a GLP-1 analog and the non-thiazolidinedione PPAR ligand are co-administered to the patient. The two compounds may be administered as separately formulated compounds or they may be administered as one formulation comprising both compounds. In a further embodiment, the stable derivative of a GLP-1 analog is administered in a regimen, which additionally comprises administration of the non-thiazolidinedione PPAR ligand. In a preferred embodiment, the stable derivative of a GLP-1 analog is a parenteral medicament. In another preferred embodiment the non-thiazolidinedione PPAR ligand is an oral medicament.

In yet another embodiment the method for treatment of type 1 diabetes, type 2 diabetes, dyslipidemia, impaired glucose tolerance, insulin resistance, obesity and/or beta-cell apoptosis comprises administration of a stable derivative of a GLP-1 analog and (-)-2-ethoxy-3-(4-(2-phenoxazin-10-yl-ethoxy)-phenyl)-propionic acid. In a still further embodiment the method for treatment of type 1 diabetes, type 2 diabetes, dyslipidemia, impaired glucose tolerance, insulin resistance, obesity and/or beta-cell apoptosis comprises administration of Arg³⁴, Lys²⁶(N^{ε}-(γ-Glu(N^{α}-hexadecanoyl)))-GLP-1 (7-37) and a non-thiazolidinedione PPAR ligand.

In yet another embodiment, the stable derivative of a GLP-1 analog and the non-thiazolidinedione PPAR ligand are administered in suboptimal dosages, i.e. dosages lower than the optimal dosages for single compound therapy.
In a further embodiment the stable derivative of a GLP-1 analog and the non-thiazolidinedione PPAR ligand are administered in sufficient amount and for a sufficient time to produce a synergistic effect, preferably for at least 4 weeks.

The subject or patient is preferably a mammal, more preferably a human.

Another aspect of the invention is a method for treatment of type 1 diabetes, type 2 diabetes, dyslipidemia, impaired glucose tolerance, insulin resistance, obesity or beta-cell apoptosis comprising administration of an effective amount of a stable derivative of a GLP-1 analog and an effective amount of 5-[[4-[3-Methyl-4-oxo-3,4-dihydro-2-quinazolinyl]methoxy]phenyl-methyl]thiazolidine-2,4-dione, or a salt thereof. 5-[[4-[3-Methyl-4-oxo-3,4-dihydro-2-quinazolinyl]methoxy]phenyl-methyl]thiazolidine-2,4-dione is disclosed in WO 97/41097.

Another aspect of the invention is a method for for increasing the number of beta-cells in a subject, increasing the size of beta-cells in a subject or stimulating beta-cell proliferation comprising administration of an effective amount of a stable derivative of a GLP-1 analog and an effective amount of 5-[[4-[3-Methyl-4-oxo-3,4-dihydro-2-quinazolinyl]methoxy]phenyl-methyl]thiazolidine-2,4-dione, or a salt thereof. In a preferred embodiment the 5-[[4-[3-Methyl-4-oxo-3,4-dihydro-2-quinazolinyl]methoxy]phenyl-methyl]thiazolidine-2,4-dione, or a salt thereof is administered in combination with Arg³⁴, Lys²⁶(N^{ε}-(γ-Glu(N^{α}-hexadecanoyl)))-GLP-1(7-37).

Another aspect of the invention is methods for the treatment of type 1 diabetes, type 2 diabetes, dyslipidemia, impaired glucose tolerance, insulin resistance, obesity or beta-cell apoptosis which method comprises administration of an effective amount of a stable derivative of a GLP-1 analog and an effective amount of an insulin sensitizer selected from pioglitazone and rosiglitazone, to a patient in need thereof. The insulin sensitizers pioglitazone and rosiglitazone are commercially available.

In one embodiment the stable derivative of a GLP-1 analog is Arg³⁴, Lys²⁶(N^{ε}-(γ-Glu(N^{α}-hexadecanoyl)))-GLP-1(7-37).

Another aspect of the invention is methods for increasing the number of beta-cells in a subject, increasing the size of beta-cells in a subject or stimulating beta-cell proliferation, which method comprises administration of an effective amount of a stable derivative of a GLP-1 analog and an effective amount of an insulin sensitizer selected from pioglitazone and rosiglitazone to a patient in need thereof.

In one embodiment the stable derivative of a GLP-1 analog is Arg³⁴, Lys²⁶(N^{ε}-(γ-Glu(N^{α}-hexadecanoyl)))-GLP-1(7-37).

In this application treatment is defined as the management and care of a patient for the purpose of combating the disease, condition, or disorder and includes the administration of the active compounds to prevent the onset of the symptoms or complications, or alleviating the symptoms or complications, or eliminating the disease, condition, or disorder.
In the context of the present application, co-administration of two compounds is defined as administration of the two compounds to the patient within 24 hours, including separate administration of two medicaments each containing one of the compounds as well as simultaneous administration whether or not the two compounds are combined in one formulation or whether they are in two separate formulations.
A medicament is a pharmaceutical composition suitable for administration of the pharmaceutically active compound to a patient.

The route of administration may be any route, which effectively transports the active compound to the appropriate or desired site of action, such as oral, nasal, buccal, pulmonal, transdermal or parenteral.

Pharmaceutical compositions (or medicaments) containing a stable derivative of a GLP-1 analog, such as Arg³⁴, Lys²⁶(N^{ε}-(γ-Glu(N^{α}-hexadecanoyl)))-GLP-1(7-37), may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of stable derivatives of GLP-1 analogs in the form of a nasal or pulmonal spray. As a still further option, the stable derivative of a GLP-1 analog can also be administered transdermally, *e.g.* from a patch, optionally a iontophoretic patch, or transmucosally, *e.g*. bucally. The above-mentioned possible ways to administer stable derivatives of GLP-1 analogs are not considered as limiting the scope of the invention.

Pharmaceutical compositions containing stable derivatives of GLP-1 analogs, such as Arg³⁴, Lys²⁶(N^{ε}-(γ-Glu(N^{α}-hexadecanoyl)))-GLP-1(7-37), may be prepared by conventional techniques, *e.g*. as described in Remington's Pharmaceutical Sciences, 1985 or in Remington: The Science and Practice of Pharmacy, 19th edition, 1995.

Thus, the injectable compositions of stable derivatives of GLP-1 analogs can be prepared using the conventional techniques of the pharmaceutical industry which involves dissolving and mixing the ingredients as appropriate to give the desired end product.

According to one procedure, e.g. Arg³⁴, Lys²⁶(N^{ε}-(γ-Glu(N^{α}-hexadecanoyl)))-GLP-1(7-37) is dissolved in an amount of water which is somewhat less than the final volume of the composition to be prepared. An isotonic agent, a preservative and a buffer are added as required and the pH value of the solution is adjusted - if necessary - using an acid, e.g. hydrochloric acid, or a base, e.g. aqueous sodium hydroxide as needed. Finally, the volume of the solution is adjusted with water to give the desired concentration of the ingredients.

Examples of isotonic agents are sodium chloride, mannitol and glycerol.

Examples of preservatives are phenol, m-cresol, methyl p-hydroxybenzoate and benzyl alcohol.

Examples of suitable buffers are sodium acetate and sodium phosphate.

Further to the above-mentioned components, solutions containing a stable derivative of a GLP-1 analog may also contain a surfactant in order to improve the solubility and/or the stability of the peptide.

According to one embodiment of the present invention, the stable derivative of a GLP-1 analog is provided in the form of a composition suitable for administration by injection. Such a composition can either be an injectable solution ready for use or it can be an amount of a solid composition, e.g. a lyophilised product, which has to be dissolved in a solvent before it can be injected. The injectable solution preferably contains not less than about 0.1 mg/ml, typically from 0.1 mg/ml to 5 mg/ml, such as from 0.5 mg/ml to 5 mg/ml of stable derivative of GLP-1 analog.

Derivatives of GLP-1 analogs such as Arg³⁴, Lys²⁶(N^{ε}-(γ-Glu(N^{α}-hexadecanoyl)))-GLP-1 (7-37) can be used in the treatment of various diseases. The optimal dose level for any patient (effective dosage) will depend on the disease to be treated and on a variety of factors including the efficacy of the specific stable derivative of a GLP-1 analog employed, the age, body weight, physical activity, and diet of the patient, on a possible combination with other drugs, and on the severity of the case.

Throughout the present application an effective dosage is defined as a dosage which is sufficient in order for the treatment of the patient to be effective. A suboptimal dosage of a pharmaceutically active compound is defined as a dosage which is below the optimal dosage for that compound when used in single-compound therapy.

Pharmaceutical compositions (or medicaments) containing non-thiazolidinedione PPAR ligands, such as (-)-2-ethoxy-3-(4-(2-phenoxazin-10-yl-ethoxy)-phenyl)-propionic acid or a salt thereof, may be administered by suitable dosage forms such as oral, nasal, pulmonal, buccal or transdermal to patients in need of such a treatment. The preferred route of administration of non-thiazolidinedione PPAR ligands is orally. Pharmaceutical compositions containing non-thiazolidinedione PPAR ligands may be prepared by conventional techniques, e.g. as described in Remington: The Science and Practice of Pharmacy, 19th Edition, Gennaro, Ed., Mack Publishing Co., Easton, PA, 1995.

Typical compositions of e.g. (-)-2-ethoxy-3-(4-(2-phenoxazin-10-yl-ethoxy)-phenyl)-propionic acid include a crystalline compound of the present invention associated with a pharmaceutically acceptable excipient, which may be a carrier or a diluent or be diluted by a carrier, or enclosed within a carrier, which can be in the form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier, which may be in the form of a ampoule, capsule, sachet, paper, or other container. When the carrier serves as a diluent, it may be solid, semi-solid, or liquid material, which acts as a vehicle, excipient, or medium for the active compound. The active compound can be adsorbed on a granular solid container for example in a sachet. Some examples of suitable carriers are water, salt solutions, alcohol's, polyethylene glycol's, polyhydroxyethoxylated castor oil, peanut oil, olive oil, gelatine, lactose, terra alba, sucrose, cyclodextrin, amylose, magnesium stearate, talc, gelatine, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art.

The pharmaceutical compositions can be sterilized and mixed, if desired, with auxiliary agents, emulsifiers, salt for influencing osmotic pressure, buffers and/or colouring substances and the like, which do not deleteriously react with the active compound.

If a solid carrier is used for oral administration, the preparation may be tabletted, placed in a hard gelatine capsule in powder or pellet form or it can be in the form of a troche or lozenge. If a liquid carrier is used, the preparation may be in the form of a syrup, emulsion, soft gelatine capsule or sterile injectable liquid such as an aqueous or non-aqueous liquid suspension or solution.

For nasal administration, the preparation may contain the compound of the present invention dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, cornstarch, and/or potato starch. A syrup or elixir can be used in cases where a sweetened vehicle can be employed.

A typical tablet of a non-thiazolidinedione PPAR ligand, e.g. (-)-2-ethoxy-3-(4-(2-phenoxazin-10-yl-ethoxy)-phenyl)-propionic acid, which may be prepared by conventional tabletting techniques, may contain:

| Core: | |
|---|---|
| Active compound | 5 mg |
| Colloidal silicon dioxide (Aerosil) | 1.5 mg |
| Cellulose, microcryst. (Avicel) | 70 mg |
| Modified cellulose gum (Ac-Di-Sol) | 7.5 mg |
| Magnesium stearate | Ad. |

| Coating: | |
|---|---|
| HPMC approx. | 9 mg |
| *Mywacett 9-40 T approx. | 0.9 mg |

| | |
|---|---|
| *Acylated monoglyceride used as plasticizer for film coating. | |

The non-thiazolidinedione PPAR ligands are effective over a wide dosage range. For example, in the treatment of adult humans, dosages from 0.01 mg/day to 10 mg/day, preferably from 0.1 mg/day to 3 mg/day may be used. A most preferable dosage is less than 2 mg/day. In choosing a regimen for patients it may frequently be necessary to begin with a dosage of from about 2 to about 10 mg per day and when the condition is under control to reduce the dosage as low as from about 0.01 to about 3 mg per day. The exact dosage will depend upon the mode of administration, on the therapy desired, the administration form, the subject to be treated and the body weight of the subject to be treated.

Generally, the non-thiazolidinedione PPAR ligands of the present invention are dispensed in unit dosage form comprising from about 0.01 to about 10 mg of active ingredient together with a pharmaceutically acceptable carrier per unit dosage.

Usually, dosage forms suitable for oral, nasal, pulmonary or transdermal administration comprise from about 0.01 mg to about 10 mg, preferably from about 0.1 mg to about 3 mg of the compound of the invention admixed with a pharmaceutically acceptable carrier or diluent.

Irrespective of the dosage forms for the stable derivative of a GLP-1 analog and for the non-thiazolidinedione PPAR ligand, they may advantageously be supplied as a kit for treatment of type 1 diabetes, type 2 diabetes, dyslipidemia, impaired glucose tolerance, insulin resistance, obesity and/or beta-cell apoptosis. The kit may contain a single dosage form or it may contain two dosage forms, i.e. one for each compound to be administered.

The combined treatment with a stable derivative of a GLP-1 analog and a non-thiazolidinedione PPAR ligand may also be combined with a third or more further pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are : Insulin, GLP-1 agonists, sulphony-lureas, biguanides, meglitinides, glucosidase inhibitors, glucagon antagonists, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as HMG CoA inhibitors (statins), compounds lowering food intake, RXR agonists and agents acting on the ATP-dependent potassium channel of the β-cells; Cholestyramine, colestipol, clofibrate, gemfibrozil, lovastatin, pravastatin, simvastatin, probucol, dextrothyroxine; β-blockers such as alprenolol, atenolol, timolol, pindolol, propranolol and metoprolol, ACE (angiotensin converting enzyme) inhibitors such as benazepril, captopril, enalapril, fosinopril, lisinopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TRβ agonists; histamine H3 antagonists.

It should be understood that any suitable combination of the compounds according to the invention with one or more of the above-mentioned compounds and optionally one or more further pharmacologically active substances are considered to be within the scope of the present invention.

### EXPERIMENTAL

Synergistic effect of combining (-)-2-ethoxy-3-(4-(2-phenoxazin-10-yl-ethoxy)-phenyl)-propionic acid and Arg³⁴, Lys²⁶(N^{ε}-(γ-Glu(N^{α}-hexadecanoyl)))-GLP-1(7-37) on glucose and HbA_{1c} (glycosylated hemoglobin) in the male ZDF rat.

### Study design:

Ninety male ZDF rats aged 15-16 weeks were used in the study. Before treatment start, measurements of glucose and HbA_{1c} were performed. All animals were overtly diabetic at the beginning of the study. Animals were allocated into the following 4 treatment groups:

| | |
|---|---|
| Group 1: | Vehicle-1 + Vehicle-2 (n=10) |
| Group 2: | (-)-2-ethoxy-3-(4-(2-phenoxazin-10-yl-ethoxy)-phenyl)-propionic acid, 1 mg/kg + Vehicle-2 (n=10) |
| Group 3: | Vehicle-1 + Arg³⁴, Lys²⁶(N^{ε}-(γ-Glu(N^{α}-hexadecanoyl)))-GLP-1(7-37), 50 µg/kg (n=10) |
| Group 4: | (-)-2-ethoxy-3-(4-(2-phenoxazin-10-yl-ethoxy)-phenyl)-propionic acid, 1 mg/kg + Arg³⁴, Lys²⁶(N^{ε}-(γ-Glu(N^{α}-hexadecanoyl)))-GLP-1(7-37), 50 µg/kg (n=10) |

(-)-2-Ethoxy-3-(4-(2-phenoxazin-10-yl-ethoxy)-phenyl)-propionic acid and Vehicle-1 were administered by oral gavage once daily at approx. 07:30. Arg³⁴, Lys²⁶(N^{ε}-(γ-Glu(N^{α}-hexadecanoyl)))-GLP-1 (7-37) and Vehicle-2 were administered subcutaneously twice daily at approx. 07:30 and 14:30.

After four weeks treatment, HbA_{1c} was measured and 24-hour glucose profiles were assessed.

### Results:

The findings of group 1, 2, 3 and 4 are listed in the table below (mean±SEM). Delta HbA_{1c} refers to the numerical difference between the measurement after treatment and the measurement before treatment. Glucose_{24hAUC} refers to the total area under the glucose concentration curve during the 24-hour period. A two-way analysis of variance was performed for each parameter and the significance of the interaction term evaluated.

| | Group 1 | Group 2 | Group 3 | Group 4 | P value of interaction term in two-way ANOVA |
|---|---|---|---|---|---|
| Delta HbA_{1c} (% points) | 1.24±0.17 | -0.81±0.36 | 0.28±0.19 | -3.78±0.18 | p<0.0002 |
| Glucose_{24hAUC} (mM×h) | 538±6 | 456±38 | 508±4 | 256±27 | p<0.001 |

The highly significant interaction terms demonstrate that four weeks combination treatment with (-)-2-ethoxy-3-(4-(2-phenoxazin-10-yl-ethoxy)-phenyl)-propionic acid (1 mg/kg, once daily) and Arg³⁴, Lys²⁶(N^{ε}-(γ-Glu(N^{α}-hexadecanoyl)))-GLP-1 (7-37) (50 µg/kg, twice daily) has synergistic (greater than additive) effects on HbA_{1c} and 24-hour glucose profiles in overtly diabetic ZDF rats.

## Claims

1. A method for the treatment of type 1 diabetes, type 2 diabetes or dyslipidemia, which method comprises administration of an effective amount of a stable derivative of a GLP-1 analog and an effective amount of a non-thiazolidinedione PPAR ligand to a patient in need thereof.

2. A method for the treatment of impaired glucose tolerance, insulin resistance, obesity or beta-cell apoptosis, which method comprises administration of an effective amount of a stable derivative of a GLP-1 analog and an effective amount of a non-thiazolidinedione PPAR ligand to a patient in need thereof.

3. A method for increasing the number of beta-cells in a subject, increasing the size of beta-cells in a subject or stimulating beta-cell proliferation, which method comprises administration of an effective amount of a stable derivative of a GLP-1 analog and an effective amount of a non-thiazolidinedione PPAR ligand to a patient in need thereof.

4. A method according to any one of claims 1-3, wherein the stable derivative of a GLP-1 analog is Arg³⁴, Lys²⁶(N^{ε}-(γ-Glu(N^{α}-hexadecanoyl)))-GLP-1 (7-37).

5. A method according to any one of claims 1-4, wherein the non-thiazolidinedione PPAR ligand is a β-aryl-α-oxosubstituted alkylcarboxylic acid, or a salt thereof.

6. A method according to any one of claims 1-5, wherein the non-thiazolidinedione PPAR ligand is (-)-2-ethoxy-3-(4-(2-phenoxazin-10-yl-ethoxy)-phenyl)-propionic acid, or a salt thereof.

7. A method according to any one of claims 1-6, wherein the stable derivative of a GLP-1 analog is Arg³⁴, Lys²⁶(N^{ε}-(γ-Glu(N^{α}-hexadecanoyl)))-GLP-1(7-37) and the non-thiazolidinedione PPAR ligand is (-)-2-ethoxy-3-(4-(2-phenoxazin-10-yl-ethoxy)-phenyl)-propionic acid, or a salt thereof.

8. A method according to any one of claims 1-7, wherein the stable derivative of a GLP-1 analog is administered in a regimen which additionally comprises administration of the non-thiazolidinedione PPAR ligand.

9. A method according to any one of claims 1-8, wherein the stable derivative of a GLP-1 analog and the non-thiazolidinedione PPAR ligand are co-administered.

10. A method according to any one of claims 1-9, wherein the stable derivative of a GLP-1 analog is a parenteral medicament.

11. A method according to any one of claims 1-10, wherein the non-thiazolidinedione PPAR ligand is an oral medicament.

12. A method according to any one of claims 1-11, wherein the stable derivative of a GLP-1 analog and the non-thiazolidinedione PPAR ligand are administrered in suboptimal dosages.

13. A method according to any one of claims 1-12, wherein the dosage of stable derivative of a GLP-1 analog is from 0.5 µg/kg/day to 5 µg/kg/day.

14. A method according to any one of claims 1-13, wherein the dosage of non-thiazolidinedione PPAR ligand is from 0.01 mg/day to 10 mg/day, preferably from 0.1 mg/day to 3 mg/day, most preferable less than 2 mg/day.

15. A method according to any one of claims 1-14, wherein the stable derivative of a GLP-1 analog and the non-thiazolidinedione PPAR ligand are administered in amounts and for a sufficient time to produce a synergistic effect.

16. Use of a stable derivative of a GLP-1 analog and a non-thiazolidinedione PPAR ligand for the preparation of one or more medicaments for the treatment of type 1 diabetes, type 2 diabetes or dyslipidemia in a patient in need thereof.

17. Use of a stable derivative of a GLP-1 analog and a non-thiazolidinedione PPAR ligand for the preparation of one or more medicaments for the treatment of impaired glucose tolerance, insulin resistance, obesity or beta-cell apoptosis in a patient in need thereof.

18. Use of a stable derivative of a GLP-1 analog and a non-thiazolidinedione PPAR ligand for the preparation of one or more medicaments for increasing the number of beta-cells in a subject, increasing the size of beta-cells in a subject or stimulating beta-cell proliferation in a patient in need thereof.

19. Use according to any one of claims 16-18, wherein the stable derivative of a GLP-1 analog is Arg³⁴, Lys²⁶(N^{ε}-(γ-Glu(N^{α}-hexadecanoyl)))-GLP-1(7-37).

20. Use according to any one of claims 16-19, wherein the non-thiazolidinedione PPAR ligand is a β-aryl-α-oxosubstituted alkylcarboxylic acid, or a salt thereof.

21. Use according to any one of claims 16-20, wherein the non-thiazolidinedione PPAR ligand is (-)-2-ethoxy-3-(4-(2-phenoxazin-10-yl-ethoxy)-phenyl)-propionic acid, or a salt thereof.

22. Use according to any one of claims 16-21, wherein the stable derivative of a GLP-1 analog is Arg³⁴, Lys²⁶(N^{ε}-(γ-Glu(N^{α}-hexadecanoyl)))-GLP-1 (7-37) and the non-thiazolidinedione PPAR ligand is (-)-2-ethoxy-3-(4-(2-phenoxazin-10-yl-ethoxy)-phenyl)-propionic acid, or a salt thereof.

23. Use according to any one of claims 16-22, wherein the stable derivative of a GLP-1 analog is administered in a regimen which additionally comprises administration of the non-thiazolidinedione PPAR ligand.

24. Use according to any one of claims 16-23, wherein the stable derivative of a GLP-1 analog and the non-thiazolidinedione PPAR ligand are co-administered.

25. Use according to any one of claims 16-24, wherein the stable derivative of a GLP-1 analog is a parenteral medicament.

26. Use according to any one of claims 16-25, wherein the non-thiazolidinedione PPAR ligand is an oral medicament.

27. Use according to any one of claims 16-26, wherein the stable derivative of a GLP-1 analog and the non-thiazolidinedione PPAR ligand are administrered in suboptimal dosages.

28. Use according to any one of claims 16-27, wherein the dosage of stable derivative of a GLP-1 analog is from 0.5 µg/kg/day to 5 µg/kg/day.

29. Use according to any one of claims 16-28, wherein the dosage of non-thiazolidinedione PPAR ligand is from 0.01 mg/day to 10 mg/day, preferably from 0.1 mg/day to 3 mg/day, most preferable less than 2 mg/day.

30. Use according to any one of claims 16-29, wherein the stable derivative of a GLP-1 analog and the non-thiazolidinedione PPAR ligand are administered in amounts and for a sufficient time to produce a synergistic effect.

31. A method for the treatment of type 1 diabetes, type 2 diabetes or dyslipidemia, which method comprises administration of an effective amount of a stable derivative of a GLP-1 analog and an effective amount of 5-[[4-[3-Methyl-4-oxo-3,4-dihydro-2-quinazolinyl]methoxy]phenyl-methyl]thiazolidine-2,4-dione or a salt thereof, to a patient in need thereof.

32. A method for the treatment of impaired glucose tolerance, insulin resistance, obesity or beta-cell apoptosis, which method comprises administration of an effective amount of a stable derivative of a GLP-1 analog and an effective amount of 5-[[4-[3-Methyl-4-oxo-3,4-dihydro-2-quinazolinyl]methoxy]phenyl-methyl]thiazolidine-2,4-dione or a salt thereof, to a patient in need thereof.

33. A method for increasing the number of beta-cells in a subject, increasing the size of beta-cells in a subject or stimulating beta-cell proliferation, which method comprises administration of an affective amount of a stable derivative of a GLP-1 analog and an effective amount of 5-[[4-[3-methyl-4-oxo-3,4-dihydro-2-quinazolinyl]methoxy]phenylmethyl]thiazolidine-2,4-dione or a salt thereof, to a patient in need thereof.

34. A method according to any one of claims 31-33, wherein the stable derivative of a GLP-1 analog is Arg³⁴, Lys²⁶(N^{ε}-(γ-Glu(N^{α}-hexadecanoyl)))-GLP-1 (7-37).

35. Use of a stable derivative of a GLP-1 analog and 5-[[4-[3-methyl-4-oxo-3,4-dihydro-2-quinazolinyl]methoxy]phenyl-methyl]thiazolidine-2,4-dione, or a salt thereof, for the preparation of one or more medicaments for the treatment of type 1 diabetes, type 2 diabetes or dyslipidemia in a patient in need thereof.

36. Use of a stable derivative of a GLP-1 analog and 5-[[4-[3-methyl-4-oxo-3,4-dihydro-2-quinazolinyl]methoxy]phenyl-methyl]thiazolidine-2,4-dione, or a salt thereof, for the preparation of one or more medicaments for the treatment of impaired glucose tolerance, insulin resistance, obesity or beta-cell apoptosis in a patient in need thereof.

37. Use of a stable derivative of a GLP-1 analog and 5-[[4-[3-methyl-4-oxo-3,4-dihydro-2-quinazolinyl]methoxy]phenyl-methyl]thiazolidine-2,4-dione, or a salt thereof, for the preparation of one or more medicaments for increasing the number of beta-cells in a subject, increasing the size of beta-cells in a subject or stimulating beta-cell proliferation in a patient in need thereof.

38. Use according to any one of claims 35-37, wherein the stable derivative of a GLP-1 analog is Arg³⁴, Lys²⁶(N^{ε}-(γ-Glu(N^{α}-hexadecanoyl)))-GLP-1 (7-37).

39. A method for the treatment of type 1 diabetes, type 2 diabetes or dyslipidemia, which method comprises administration of an effective amount of a stable derivative of a GLP-1 analog and an effective amount of an insulin sensitizer selected from pioglitazone and rosiglitazone, to a patient in need thereof.

40. A method for the treatment of impaired glucose tolerance, insulin resistance, obesity or beta-cell apoptosis, which method comprises administration of an effective amount of a stable derivative of a GLP-1 analog and an effective amount of an insulin sensitizer selected from pioglitazone and rosiglitazone, to a patient in need thereof.

41. A method for increasing the number of beta-cells in a subject, increasing the size of beta-cells in a subject or stimulating beta-cell proliferation, which method comprises administration of an effective amount of a stable derivative of a GLP-1 analog and an effective amount of an insulin sensitizer selected from pioglitazone and rosiglitazone to a patient in need thereof.

42. A method according to any one of claims 39-41, wherein the stable derivative of a GLP-1 analog is Arg³⁴, Lys²⁶(N^{ε}-(γ-Glu(N^{α}-hexadecanoyl)))-GLP-1 (7-37).

43. Use of a stable derivative of a GLP-1 analog and an insulin sensitizer selected from pioglitazone and rosiglitazone for the preparation of one or more medicaments for the treatment of type 1 diabetes, type 2 diabetes or dyslipidemia in a patient in need thereof.

44. Use of a stable derivative of a GLP-1 analog and an insulin sensitizer selected from pioglitazone and rosiglitazone for the preparation of one or more medicaments for treatment of impaired glucose tolerance, insulin resistance, obesity or beta-cell apoptosis in a patient in need thereof.

45. Use of a stable derivative of a GLP-1 analog and an insulin sensitizer selected from pioglitazone and rosiglitazone for increasing the number of beta-cells in a subject, increasing the size of beta-cells in a subject or stimulating beta-cell proliferation in a patient in need thereof.

46. Use according to any one of claims 43-45, wherein the stable derivative of a GLP-1 analog is Arg³⁴, Lys²⁶(N^{ε}-(γ-Glu(N^{α}-hexadecanoyl)))-GLP-1 (7-37).
